(19) Europäisches Patentamt
European Patent Office
Office européen des brevets

(11) **EP 4 385 470 A1**

(12) **EUROPEAN PATENT APPLICATION**

(43) Date of publication:
**19.06.2024 Bulletin 2024/25**

(21) Application number: **22213252.4**

(22) Date of filing: **13.12.2022**

(51) International Patent Classification (IPC):
**A61F 9/008** (2006.01)     **A61B 5/00** (2006.01)

(52) Cooperative Patent Classification (CPC):
**A61F 9/00821; A61B 5/0095; A61B 5/01**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **OD-OS GmbH**
**14513 Teltow (DE)**

(72) Inventors:
• **Paulauskas, Gediminas**
  **12165 Berlin (DE)**
• **Friede, Sebastian**
  **10781 Berlin (DE)**
• **Mundt, Arne**
  **14476 Potsdam (DE)**

(74) Representative: **Pfenning, Meinig & Partner mbB**
**Patent- und Rechtsanwälte**
**Joachimsthaler Straße 10-12**
**10719 Berlin (DE)**

(54) **TREATMENT DEVICE AND MEASURING DEVICE WITH A MOBILE MEASURING UNIT**

(57) The invention refers to the measurement of tissue temperature or another control value during the treatment of biological tissue, in particular tissue in the eye (5) of a patient, by a therapy laser (1). During the treatment, in addition to the pulses that are intended for the therapeutical heating of the tissue, temperature measurement signals or more generally, measurement signals for a control value (dedicated laser signals) (16a, 16b) are generated. A measuring device is provided with a mobile measuring unit (7) comprising a pressure converter (7a, 8a, 11, 12) which is arranged to detect the opto-acoustic pulses generated by the dedicated laser signals for determining a control value, e.g., the temperature of the tissue. The mobile measuring unit comprises a pressure converter (7a, 8a, 11, 12) for measuring transient pressure signals, a signal pre-processing unit (7f) and a data transmitting unit (7b) for wireless data transmission and wherein the receiving unit (7c) is arranged for the wireless reception of data. No more cables are required for the connection of the mobile measuring unit and thereby, measurement problems and errors can be significantly reduced.

Fig. 2

**Description**

The invention refers to the measurement of a control value, for example the temperature, of biological tissue, for example during treatment of the tissue, in particular in the area of ophthalmology.

It is known from the prior art that in ophtalmology, energy sources, in particular therapy lasers, are used for treatment of diseases. The laser energy is absorbed by the biological tissue, for example, at the fundus of the eye of a patient and converted to heat, whereby, as the result of the treatment, a reaction of the tissue is produced. In the case of transpupillary thermotherapy, the temperature increase is utilized for achieving a vascular occlusion. For photodynamic therapy, a previously injected dye is activated by laser irradiation and develops its effect on the cells to which it is bound. In this case, the energy of the therapy laser is absorbed in the dye and in the retina. By the therapy laser application, a temperature increase of the biological tissue is achieved. For example, by laser photocoagulation, parts of the retina of the eye can be thermally coagulated. In order to achieve the desired effects, in some cases, temperatures of above 60° C can be reached. For the success of an ophthalmological treatment, in many cases the measurement and control of the tissue temperature is key.

In the German patent specification DE 10135944C2 and a corresponding US patent document US2003/0032949A1, respective ophthalmologic treatments are described as well as a method for temperature measurement, wherein the temperature measurement comprises the application of radiation pulses to the tissue and the detection and analysis of photo- acoustic signals which are the result of tissue expansions and/or contractions. The pressure wave signals that occur as the result of the photo-acoustic effect are detected by a pressure converter in a contact lens which is brought in contact with the eye of the patient. These signals depend on the temperature of the tissue and hence, allow for the determination of the temperature.

Considering the background of the prior art, it is a goal of the current invention to improve the quality of control by measuring a control value, for example the temperature and to improve the signal to noise ratio during signal processing. This is important because of the high volume of data of the transient pressure waves that has to be measured and recorded.

The invention refers to a measuring device for measuring a control value, in particular the tissue temperature in the eye of a patient with an excitation source for pulse-like or edge- like excitation of the tissue by means of a beam falling through the eye lens, in particular a laser beam or laser signal, with a mobile measuring unit for placing on the patient's eye for the detection and/or measurement of opto-acoustic pulses or pressure waves, and with a receiving unit for receiving data from the mobile measuring unit, wherein the mobile measuring unit comprises a pressure converter for measuring transient pressure signals, a signal pre-processing unit and a data transmitting unit for wireless data transmission and wherein the receiving unit is arranged for the wireless reception of data.

In the context of this application, the expression "pulse like signal" may always comprise edge-like signals with a rising or falling flank, because this kind of shape of a signal may, in the same way as pulses with a rising and a falling flank, generate pressure waves.

It should be understood that in the context of this application, the detection and/or measurement of opto-acoustic pulses also includes and may mean the detection or measurement of pressure wave signals that are the consequence or that represent the acoustic effect of the optical signals or pulses.

From the prior art, it is known to connect a pressure converter in a contact lens to a data processing unit by movable cables. This creates additional effort during the use of the device at a patient due to the cables that are disturbing when the contact lens has to be approached to the patient's eye or vice versa. Further, when the cables are touched during measurement, the signals are electrically disturbed given the high frequency range where the measurement of the transients takes place.

At a first glance, the choice of a wireless connection for transmission of the transient wave data seems to be critical due to the high bandwidth that is needed. But it emerged, that the necessary bandwidth can be delivered, that appropriate data transfer protocols or other ways of preparing the data before transfer are available and in addition, a preprocessing of the data in the contact lens, which is now formed as a mobile measuring unit, can help to reduce the amount of data that has to be transferred and the wireless connection, after all, is less prone to failure and disturbances than a cable transmission. The mobile measuring unit can then be movable freely and at the same time, the quality of data that arrive at the receiving unit is improved.

The invention may further refer to a treatment device for the treatment of biological tissue, in particular tissue in the eye of a patient, by radiation, with a radiation source, in particular a therapy laser, for targeted heating of the tissue with the aim of generating a tissue reaction, and with a measuring device of the kind mentioned above.

By combining a measuring device, in particular a temperature measuring device of the kind described above with a device for the treatment of biological tissue, full use can be made of the efficiency and accurateness of the measurement of a control value, for example a tissue temperature measurement during the treatment, in particular during ophthalmologic applications. Both the control value and/or the measured temperature may be used to control the power of the therapy laser with the goal to achieve or to not exceed a target tissue temperature. The control value may generally comprise a parameter representing the current tissue temperature, but it may also and/or in addition comprise a parameter representing the absorption strength of the laser light (which is applied for the therapy and potentially also for the measurement) in the tissue at the fundus of the eye. Hence, also the intensity of the pigmentation at the place where the treatment currently takes place may have influence on the control value.

The invention may be implemented by that the excitation source is identical to the radiation source, in particular a therapy laser.

By choosing the same source for the excitation of tissue for the purpose of the measurement of a control value and for the therapy, a cost reduction can be achieved. Even more important may be in some cases that the laser beam for excitation of the opto-acoustic pressure waves and for the treatment can be directed and controlled by the same optical arrangement with lenses and mirrors with the same laser and the same laser control. Hence, it can easily be made sure that the control value, e.g., the temperature, is measured at the same place or in the same range where the therapy takes place. In cases where it should be avoided that the excitation beam hits the same place where a therapy takes place, this can also easily be guaranteed.

The invention may further refer to a mobile measuring unit for a measuring device according to the kind mentioned above for placing on the patient's eye, which comprises a pressure converter for detecting transient pressure wave signals and a signal preprocessing unit, a data transmitting unit for wireless data transmission and an antenna.

The mobile measuring unit may further be implemented in the way that the signal preprocessing unit comprises at least one, two, three, four, five, six or seven of the following elements: an amplitude modulator or a frequency modulator for modulating the measured signal on a radio wave, a signal amplifier, a band pass filter, a high pass filter, a low pass filter, a unit for digitizing data or signals, a unit for integrating over signals, a unit for averaging over signals or data.

The amplitude modulator or a frequency modulator for modulating the measured signal on a radio wave may establish the possibility to send the measured signals, which are preprocessed by modulating them to radio waves, in analogue, wireless form to a stationary receiver. This transmission method is well established and not prone to disturbances on a short distance.

Band pass filters, high and low pass filters allow for the optimized processing of signals. The integration over signals and the averaging over several signals allow for a reduction of noise and measurement errors and help improving the signal to noise ratio. If the excitation of opto-acoustic pulses/signals is regular with a fixed frequency, a lock-in amplifier can be used for the processing of measurements.

By the preprocessing of data by integration and/or averaging, the data volume that has to be transmitted from the mobile measuring unit to the receiving unit is drastically reduced. If the signals are transformed into digital data by an analogue to digital converter, the usual standards for a wireless digital data transmission can be used.

The mobile measuring unit may further comprise rechargeable energy storage means.

As the mobile measuring unit has no connection to a base station by a cable, it has to be autonomous with regard to energy supply and therefore needs a source of energy, e.g., a battery or an accumulator which should be rechargeable.

For recharging, energy can be provided to the mobile measuring unit and/or to the accumulator inside the mobile measuring unit by any form of energy transfer by cable or by wireless energy transfer, e.g. by wireless energy transfer via electric/magnetic fields or by electromagnetic waves or by energy harvesting of mechanical movements by a dynamo element or of light by a light sensitive element.

The mobile measuring unit may further comprise a device for overvoltage protection and/or for short-circuit protection and/or for protection against incorrect polarity.

By these features, errors and misappropriate connection to external power sources can be avoided or at least potential damages caused by such misappropriate connections can be avoided.

Another form of implementation of the invention may provide a disposable contact element for establishing the physical contact to the patient's eye, which is or can be fixed on a front side of the tubular housing or of a pressure wave transfer body by a snap-in mechanism in such a way that it is in steady contact with a carrier of the pressure converter, in particular a pressure wave transfer body. The pressure wave transfer body itself may be formed by a part of the tubular housing or it may be integrated into the housing.

Such a contact element may be disposed of after use and for each patient, a new contact element will be installed/snapped on. Therefore, it is helpful if the contact element can simply be snapped in or on e.g. the housing wall of the housing of the mobile measuring unit. The snap-in mechanism can be designed in a way that the contact element is steadily pressed against the carrier of the pressure sensor by an elastic force or element, so that a certain minimal contact and/or pressure force is kept, and pressure waves may easily be transferred between the contact element, the carrier/pressure wave transfer body and the pressure converter. By establishing a defined stable pressure force between the contact element and the carrier, the transfer of the pressure waves is less dependent on the force with which the mobile measuring unit is pressed against the eye of the patient.

The contact element may consist of a simple ring, or it may comprise a ring of which the central opening may be covered by a transparent element wherein the transparent element may be a flat glass or plastic or an optical lens which can be pressed against the eye of the patient.

For the mobile measuring unit according to the invention it may further be provided that one, two, three or more than three of the following elements are placed inside a tubular housing of the mobile measuring unit: a signal preprocessing unit, a data transmitting unit, a device for overvoltage protection, a device for short-circuit protection, a device for protection against incorrect polarity, a rechargeable energy storage means.

A mobile measuring unit according to the invention may further in one potential implementation preferably be implemented in the way that an antenna for data transmission is provided inside the tubular housing or on the outside of the tubular housing of the mobile measuring unit or in an opening of the tubular housing or that an antenna is formed by an opening of the tubular housing, wherein the antenna is connected to the data transmitting unit.

For an effective and efficient wireless connection of the mobile measuring unit with the stationary receiving unit, a good wireless connection is needed and therefore, good conditions for data transmission and reception are important. In order to secure a good data transmission, a good and effective antenna in or at the mobile measurement unit is important.

In case the tubular housing allows for the transmission of electromagnetic signals through its walls, an antenna may be placed inside the tubular housing. This may be the case when the tubular housing is made of plastic or resin or glas or ceramics or any material that is not electrically conductive.

In cases where the tubular housing is made of metal or is electrically conductive, an antenna on the outer wall of the housing or in an opening can be used or an antenna can be formed by an opening in the housing, e.g., by a slit antenna. If the antenna is formed on the outside of the housing, it may e.g., be printed as an insulated conductive layer on the outside.

For further implementations of the invention and for more detailed features, the invention is shown in drawings and explained below.

Therein, the figures of the drawings are showing:

Figure 1:     a setup for ophthalmologic laser treatment with temperature monitoring according to the prior art,

Figure 2:     a setup for ophthalmologic laser treatment with a temperature measuring device including a mobile measuring unit,

Figure 3:     the internal structure of a mobile measuring unit,

Figure 4:     a structure of an antenna,

Figure 5:     a slit antenna in the housing of the mobile measuring unit,

Figure 6:     absorption spectra of different biological materials and laser emission wavelengths of different lasers,

Figure 7-12:  different patterns of therapy laser pulses and laser pulses intended for temperature measurement,

Figure 13:    a setup for laser treatment and a temperature measurement with a single laser and with a mobile measuring unit for the detection of pressure waves,

Figure 13a:   a part of a measuring unit with a disposable contact element,

Figure 13b:   a part of a measuring unit with another implementation of a disposable contact element,

Figure 14:    a cross section of a mobile measuring unit as schematically shown in Figure 13,

Figure 15:    a view inside the mobile measuring unit as indicated by the dotted line in Figure 14,

Figure 16:    the internal structure of a charging station for a mobile measuring unit,

Figure 17:    the internal structure of a mobile measuring unit,

Figure 18:    a perspective view of a pressure converter in the form of a piezo ring which, on its bottom side, shall be connected to a pressure wave transfer body,

Figure 19:    schematically the pressure converter of figure 18 and the way it is fixed/connected to a pressure wave transfer body,

Figure 20:    the relation between the mean eigenfrequency of the ring and the inner diameter of the ring as shown in figure 18 in case its bottom side is connected to a pressure wave transfer body, in a way as shown in fig. 19,

Figure 21:    the relation between the mean eigenfrequency of the ring and the thickness of the ring as shown in figure 18 in its axial direction in case its bottom side is connected to a pressure wave transfer body, in a way as shown in fig. 19,

Figure 22:    a perspective view of a pressure converter in the form of a piezo ring,

Figure 23: schematically the pressure converter ring of figure 22 and the way its rim/outer circumference is fixed/connected to a pressure wave transfer body,

Figure 24: the relation between the mean eigenfrequency of the ring and the thickness of the ring as shown in figure 22 in its axial direction in case its rim/outer circumference side is connected to a pressure wave transfer body, as shown in fig. 23,

Figure 25: a cross sectional view of a pressure wave transfer body with a pressure converter,

Figure 26: a cross sectional view of another pressure wave transfer body with a pressure converter and

Figure 27: a cross sectional view of a pressure converter in the form of a toroid.

In many areas of ophthalmology, different energy sources, particularly lasers, are used for diagnostics and treatment. As a rule, the entire beamed-in energy is absorbed by the biological tissue and converted to heat, the resulting temperature increase achieving the desired treatment effect. For example, during laser photocoagulation, the retina of the eye is thermally coagulated in a targeted manner. In the case of the conventional irradiations with irradiation times about 100 ms, temperatures of above 60° C occur. Also, in the case of transpupillary thermotherapy (TTT), temperature increases are utilized for achieving a vascular occlusion. In the case of photodynamic therapy (PDT), a previously injected dye is activated by laser irradiation of a therapy laser on the ocular fundus. The active ingredient develops its effect only on those cells to which it is bound. In this case also, almost the entire beamed-in energy is absorbed in the dye and in the retina and is converted to heat. During the respective irradiation time (pulse duration) with relatively long treatment and radiation pulses in the order of from $\mu$s to several hundred seconds, a temperature increase of the treated biological tissue, particularly of the fundus of the eye, which results in unintended damage to regions of the retina, should in any case be avoided. A non-invasive real-time temperature determination during ophthalmologic laser treatment has been desired for a long time. As a solution, a technology has been developed which allows to monitor the tissue temperature during the thermal treatment of biological tissue, in particular during ophthalmologic treatments, by means of optoacoustic techniques. In more detail, it is known to determine the tissue temperature of biological tissue caused by a laser treatment with optoacoustic techniques by pulsed laser irradiation. Temperatures are measured at the ocular fundus for example during the selective microphoto coagulation or during any other treatment of diseases of the retina which uses an irradiation source like, for example, a therapy laser. A special laser pulse which is intended for temperature measurement generates a thermal tissue expansion or, at the end of the pulse, a thermal tissue contraction, wherein the expansion as well as the contraction generate pressure waves and wherein the amplitude or other characteristics of the pressure waves can be used for calibrating the pressure wave characteristics, e.g., the amplitude, to the temperature or to a more complex control value with a defined relation to the temperature and to an absorption characteristic of the ocular fundus. From the change of subsequent pressure wave characteristics after subsequent treatment pulses, the temperature increase or decrease and, in addition, the respective absolute temperatures can be determined by means of the relation between the temperature of the tissue and the pressure wave characteristics, in particular the pressure wave amplitude, which is defined by the Grüneisen coefficient. It is an object of the current invention to provide a simplified method and a system of the kind described above for the non-invasive determination of the control value mentioned above or of the temperature on treated biological tissue, which can also be an implementation of a control value. The control value or the measured temperature may then, as already explained above, serve for controlling the power of the therapy laser. The control value may for example be the current temperature of the treated tissue of the eye, which can be determined based on the measurement of the strength of the opto- acoustic pulses or the detected pressure waves. For example, the strength of the pressure waves, their amplitude, average amplitude or accumulated energy may be measured or determined.

The measured strength of the pressure waves depends on the tissue temperature, in which the pressure waves are generated, the absorption of the dedicated laser signals on their way to the tissue and the intensity of absorption of the laser signals in the tissue (hence, for example: the pigmentation strength). In the range of parameters, where the treatment generally takes place, a variation of the strength of the detected pressure waves dP is determined by the following equation:

$$dP = \mu \, \Gamma \, dH,$$

wherein dP is the Variation of the strength of the pressure waves, $\mu$ ist he absorption strenght of laser light in the tissue, $\Gamma$ is the Grüneisen coefficient and dH is the accumulated laser beam energy in the tissue (heat dissipation in the tissue has been neglected for short time ranges). Because both parameters, the tissue temperature and the absorption strength of the tissue, point into the same direction (high temperature and high absorption strength require less power of the therapy laser), the measured strength of the pressure waves is an appropriate control value for controlling the power of the therapy laser. Therein, it is one option to use the control value as such for the control of the therapy laser, but it is another option to first determine the current temperature of the tissue, for example based on a calibration, and after that to control the therapy laser based on the determined temperature value.

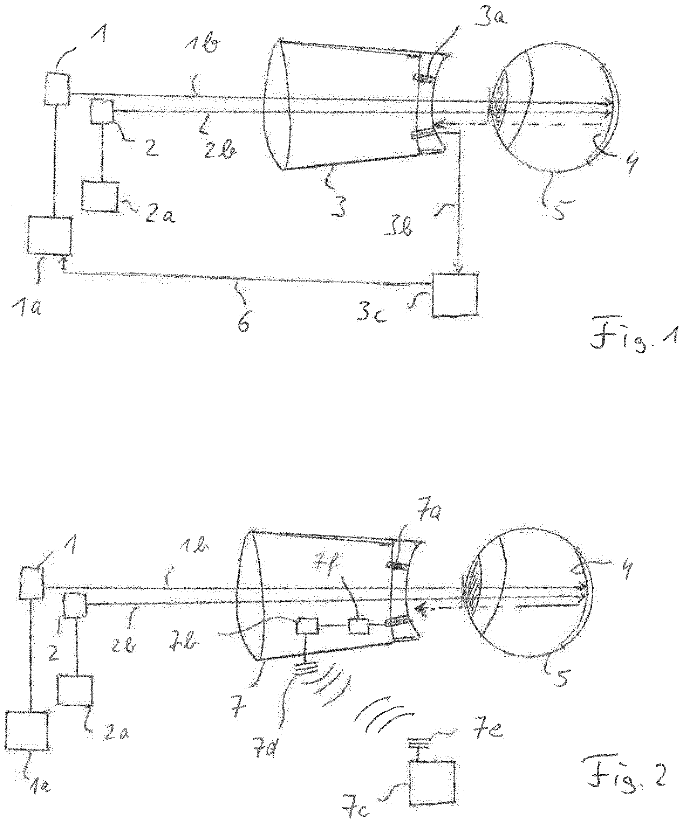

Figure 1 shows a setup with a therapy laser 1 which is controlled by a therapy laser control unit 1a and a measuring device which can be a temperature measuring device with an excitation source which is formed by an excitation laser 2 with a control unit 2a for the excitation laser 2 and a measuring unit 3 for measuring transient pressure waves.

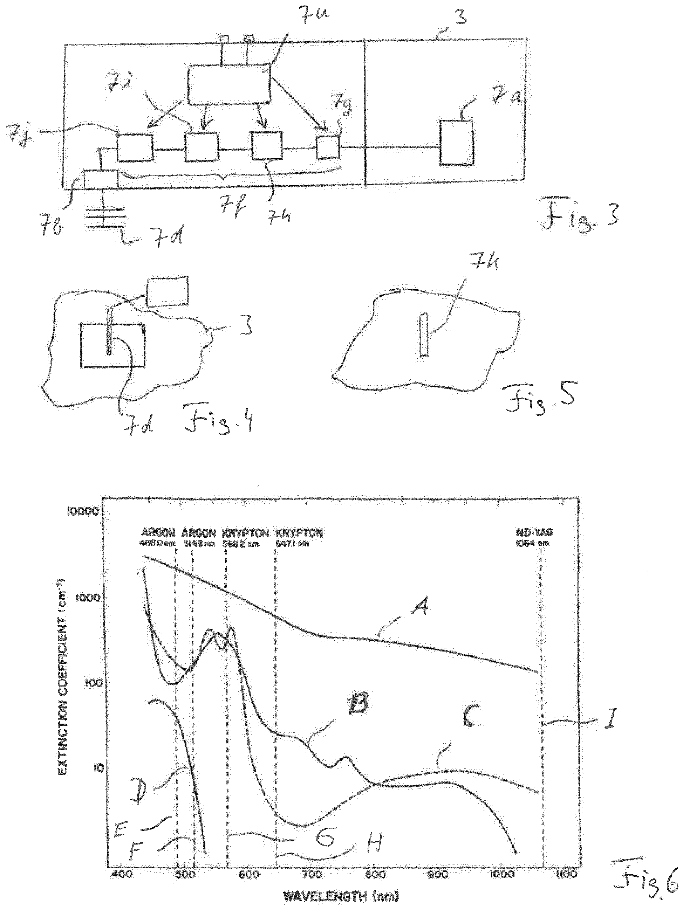

The therapy laser 1 generates a therapy laser beam 1b which is controlled to heat the biological tissue at the fundus 4 of the eye 5 of a patient. The characteristics of the therapy laser beam can be chosen appropriately dependent on the treatment that is needed. The therapy laser beam may have the form of a continuous wave or of repeated pulses or any other form and different laser types can be used according to the disease that shall be treated and the wavelength of the radiation that shall be provided and that is absorbed in the eye. An overview over some laser types that are available is given in Figure 6 and described below.

The excitation laser 2 is controlled to generate a laser beam 2b forming signals that generate thermic expansions and/or contractions in the or close to the target area of the therapy laser in the eye. The thermic expansions and/or contractions generate pressure waves that are sent out from the biological tissue through the eye 5 and can be detected by a measuring unit 3 for measuring transient pressure waves. The pressure waves can be detected by a transducer/pressure converter 3a and transformed in electrical signals, which are then sent through cables 3b to a signal processing unit 3c. The signal processing unit 3c can derive a current control value or, more specific, a temperature value from the signals that are detected by the transducer or pressure converter 3a. If the control value is not exactly the tissue temperature, it may be a value which is related to the tissue temperature and an absorption strength in the tissue of the laser light forming the dedicated laser signals of the eye. More in detail, a variation of the control value may directly be proportional to a variation of a product of the tissue temperature and the absorption strength in the tissue of the laser light forming the dedicated laser signals. The calculated temperature or the control value or its variation may for example, serve to control the therapy laser power appropriately. For this purpose, a feedback loop connection 6 can be provided between the signal processing unit 3c and the therapy laser control unit 1a.

The transient pressure wave signals that are measured by the transducer/pressure converter 3a are very high frequent signals. Therefore, the transmission of these signals over cables 3b is prone to a lot of disturbances creating errors in the determination of temperatures. This is even enhanced by any potential movement of the cables, which is difficult to avoid when the unit 3 and/or the patient is moving. Further, the cables can disturb the installation whenever the whole treatment device is set up for use.

Figure 2 therefore is showing a new setup with a mobile measuring unit 7 which comprises a data transmitting unit 7b for wireless data transmission and an antenna 7d. On the receiving side, a receiving unit 7c is provided separately from the mobile measuring unit 7. The receiving unit 7c is connected to an antenna 7e for the wireless reception of signals/data.

The wireless transmission of data, in particular of digitized or otherwise preprocessed data, is less prone to disturbances than the transmission by movable cables.

The reliability of the signal/data transmission can be enhanced by the preprocessing of the transient pressure wave signals on the side of the mobile measuring unit 7. For this purpose, a preprocessing unit 7f is provided.

A preprocessing unit 7f may comprise or be formed by a unit for modulating the measured signals on a radio wave, e.g. in the form of amplitude modulation or frequency modulation. The preprocessing unit may also comprise or be formed by an analog to digital converter and use a digital transmitter and a standardized protocol for digital data transfer like Bluetooth, Wifi, Zigbee, ethernet or any other standard.

The preprocessing unit may further comprise different elements, for example, a high pass filter and/or a low pass filter and/or a bandpass filter, an amplifier, an analog/digital converter, a unit for integrating a signal over time and an averaging unit that can determine average values from several results. A concrete example for the structure of a preprocessing unit will be shown in another figure and described below.

An antenna 7d for wireless communication can be formed in various ways. It can for example be a simple rod that is integrated in the wall of a tubular housing of the mobile measuring unit. The housing may be made of a nonconductive material and in this case, the antenna may simply be positioned inside the housing. If the housing is electrically conductive, the antenna shall be isolated from the housing. The antenna can protrude from the housing, or it can be printed as a conductive layer on the outside of the housing. If the housing is conductive, an isolating interlayer must be provided.

The antenna can also be provided in an opening of the housing, or it can be formed, in case the housing is made of a conductive material, as an opening, for example as a slit, which in this case must be coupled to the transmitter appropriately.

The transducer or pressure converter for sensing/detecting the pressure waves may also have one of several different forms which are in any form sensitive to pressure waves and change a physical parameter if they are hit by a pressure wave. Well known forms of such sensors are dielectric materials which change their dielectric constant or, in other words, their refractive index if they are hit by a pressure wave. The influence of the changing refractive index on light that passes the sensor can be measured. One of those sensors is for example an interferometer. Another kind of sensor for pressure waves are piezoelectric elements. The pressure converters 3a, 7a in figures 1 and 2 are just examples. A concrete implementation is described below.

Figure 3 shows an example of the structure of a preprocessing unit 7f which is coupled to a pressure converter 7a and a transmitter 7b. The mobile measuring unit 3 is represented schematically as a box. The preprocessing unit 7f is comprising a filter 7g, a signal integrator 7h, an averaging unit 7i and an analogue to digital converter 7j. From the pressure converter 7a, signals are sent to the filter7g, the filtered signals are integrated in the signal integrator 7h and after several laser pulses, an average is calculated over the integrated signals for all photo-acoustic pressure waves by the averaging unit 7i. In more detail, for each transient pressure wave signal, the signal is integrated and an average is calculated from the single integrated values. After digitization of the average value in the A/D converter 7j, the digital data are transmitted wirelessly over the antenna 7d by a transmitting unit 7b. A battery 7u serves as rechargeable energy storage means and may be connected with all elements of the mobile measuring unit which need energy supply.

In Figure 4, a rod shape antenna is shown which is positioned in an opening of the metallic housing 3. Figure 5 shows an antenna 7k in the form of a slit opening in a metallic housing.

In Figure 6, four different absorption curves (extinction coefficients) are shown for different materials between 400 and 1100 nanometer. Curve A represents melanin, curve B reduced hemoglobin, curve C: oxygenated hemoglobin and curve D: macular xanthophyll.

The five vertical lines show the specific emission wavelengths of different laser models: Line E is representing an Argon laser with a wavelength of 488 nm, line F an Argon laser at the wavelength of 514,5 nm, line G a Krypton laser at a wavelength of 568,2nm, line H a Krypton laser at a wavelength of 647,1 nm and line I a NDYAG laser at a wavelength of 1064 nm. The appropriate therapy laser must be chosen dependent on the intended therapy and the substance that shall absorb the laser light and hence dependent on the absorption curve of the substance.

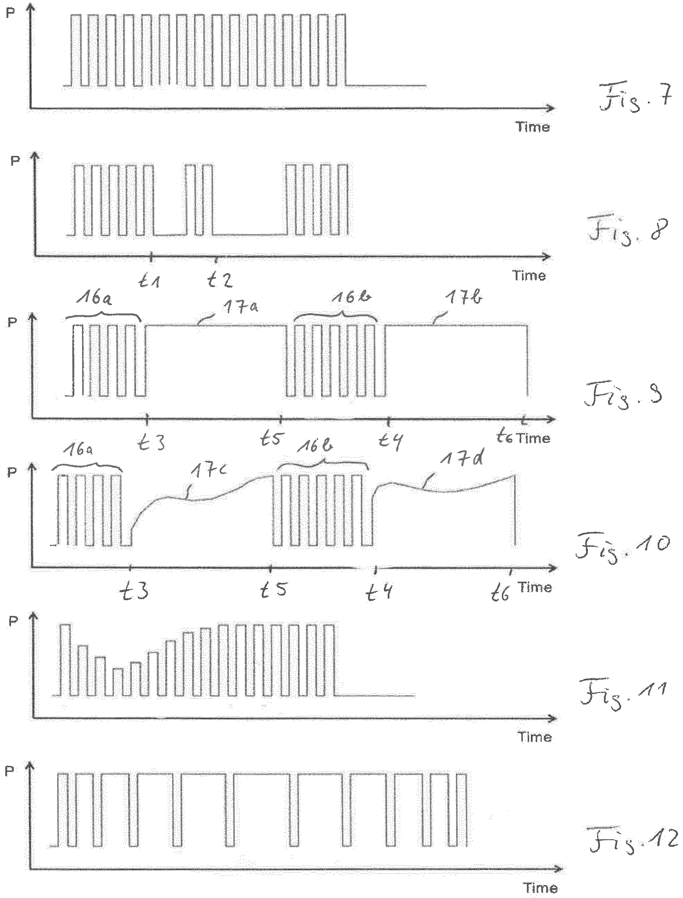

In figures 7-12, different patterns of laser activity of a therapy laser are shown over time, wherein the therapy laser delivers not only laser signals for therapeutic treatment, but also dedicated laser signals.

On the vertical axis, the laser intensity, e.g., the laser power is shown while the horizontal axis represents the running time. The diagrams are not calibrated. They shall just serve to explain the principles of the aligned laser activities wherein on one hand the therapy laser signals serve to heat biological tissue for a medical treatment and on the other hand the dedicated laser signals generate opto-acoustic laser pulses for the purpose of temperature measurement or for the purpose of determining a control value. The relation of the duration of the single pulses which shall do the medical treatment and the pulses for generation of opto-acoustic signals (dedicated laser signals) are just examples and shall in no way limit the scope of the claimed invention. The same holds for the representation of pulse pauses between the laser pulses, which are described to have a certain length even if the relation of pulses and pauses in the graphics may not be represented to scale.

Of course, the laser activity which is dedicated to the generation of opto-acoustic pulses (dedicated laser signals) can also contribute to a heating of the tissue and if this contribution is considerable, it must be taken into account when the laser activity for the treatment as such is determined or controlled. The time intervals are described using the unit "$\mu$s", which shall mean throughout this text the same as the expression "microsecond".

The laser activities in figures 7 to 12 may represent laser pulses of both kinds, that is to say, laser pulses for medical treatment and laser pulses for the generation of opto-acoustic pressure waves for temperature measurement or for the determination of a control value which is not exactly the tissue temperature, wherein both kinds of pulses may be generated by one and the same laser (a therapy laser), but the graphics may also be interpreted as showing pulses from at least two different lasers, i.e. a therapy laser and a separate laser for the generation of dedicated laser signals, which are combined so that the laser beams of both or all lasers are directed to the same tissue. In this case, it would be an advantage to combine the control of all lasers that are used in combination.

Generally, the duration of both kinds of laser signals (dedicated laser signals and laser signals which only serve for the therapy) and the steepness of the flanks of the pulses can be controlled by a laser control unit electronically or by an optical switch. A mechanical chopper may also be used. If optical switches are used, it may be an advantage, if the laser beams pass a transparent body, like a glass or plastic body, e.g. an optical fiber, wherein a parameter of the material can be controlled to control and switch on and off the optical beam which passes the material. This way, an optical switch may be formed as is generally known from the prior art.

Figure 7 shows a continuous pulse train of micro-pulses with a duration of 1 $\mu$s - 2 $\mu$s, or 25 $\mu$s - 50 $\mu$s wherein, in both cases, at least 25 $\mu$s pulse pauses are provided between the individual pulses. Each pulse is used for an incremental heating and a selected number of pulses, for example a selected number of subsequent pulses, or every second or third or tenth pulse, may also be dedicated laser signals and hence, be used for the generation and detection of opto-acoustic transients/pressure wave signals. The measured pressure wave signals may be integrated and averaged (e.g. average of 5 to 25 or 5 to 50 measurements).

Generally, a laser pulse may be defined as a dedicated laser pulse or dedicated laser signal, if it is actually used to measure a control value or in particular the tissue temperature, that is, if the opto- acoustic pulse ore pressure wave transient generated by the laser pulse is actually detected and used for determining a control value as mentioned above or in particular the tissue temperature.

Figure 8 shows an uninterrupted pulse train of micropulses (0,1 $\mu$s - 2 $\mu$s, 1 - 2 $\mu$s or 25 $\mu$s - 50 $\mu$s) wherein, in both cases, at least 25 $\mu$s pulse pauses are provided between the individual pulses. Each pulse is used for an incremental heating and for the measurement of opto-acoustic transients. The measured pressure wave signals may be averaged (5 to 50 averages). It can also be decided that only every second or third or any other selection of the pulses is used for temperature measurement or for the determination of a control value. If a target temperature of the tissue or a target control vlue is reached, the duration of the pauses between the pulses may be increased, as shown at the times designated as t1 and t2, for letting cool down the tissue by heat diffusion. The pulse pauses may be decreased or the duration of the pulses or a selection of pulses or a series of pulses may be prolongated if the temperature or the control value falls below a threshold of the target control value or the target temperature for heating up the tissue.

Figure 9 shows continuous pulse trains 16a, 16b of micro-pulses (1 $\mu$s - 2 $\mu$s) with at least 25 $\mu$s pulse pause between the individual pulses that are used for the temperature measurement and which therefore are dedicated laser signals 16a, 16b. Heating pulses 17a, 17b of a longer duration (50 $\mu$s - 20 ms), starting at times designated as t3 and t4, may be applied between the measurement pulses (dedicated laser signals). After a heating period, shorter measurement pulses (dedicated laser signals) 16b, starting at t5 and t6, are applied for determining the applied dose. The pulse length of the following heating pulse may be altered in order to heat the tissue slightly or to a larger extent. The heating period may be skipped if it is necessary to let the tissue cool down.

Figure 10 shows a continuous pulse train of micro-pulses (1 $\mu$s - 2 $\mu$s) 16a with at least 25 $\mu$s pulse pauses between the pulses, which are used for the temperature determination (dedicated laser signals). It can also be decided that the pauses between the pulses may be shorter than 25 $\mu$s. Heating pulses 17c, 17d of a duration between 50 $\mu$s and 20 ms may be applied between the measurement pulses (dedicated laser signals) 16a, 16b. The power of the heating pulses may be modulated or adapted in order to heat the tissue to an extent which is appropriate to reach the target temperature of the tissue. After each heating period, shorter measurement pulses are applied for determining the tissue temperature. The duration of the heating pulses may be more than two times, more than five times or more than ten times the duration of the measurement pulses. Each heating period may be skipped for cooling the tissue if the tissue temperature is higher than the target temperature and/or the control value is higher than a target control value Figure 11 shows a continuous pulse train of micro-pulses (1 $\mu$s - 2 $\mu$s, or 25 $\mu$s - 50 $\mu$s) with at least 25 $\mu$s pulse pauses between the individual pulses. Each pulse is used for an incremental heating and can also be used for the measurement of opto-acoustic transients if the respective pressure wave signals are detected. The measured signal may be integrated for each single pressure wave/opto-acoustic pulse and averaged (average over 5 to 25 or 50 measurements). The power of the pulses may be controlled/adapted in the form of an amplitude modulation for a control of the heating. Smaller amplitudes of the laser intensity slow down the heating or may lead to a cooling down of the tissue.

This might be useful for heating the tissue close to treatment limit temperatures or for cooling the tissue slowly. For a faster cooling, a pulse sequence may be skipped.

Figure 12 shows a continuous pulse train of micro-pulses (1 $\mu$s - 2 $\mu$s, or 25 $\mu$s - 50 $\mu$s) with at least 25 $\mu$s pulse pause between the individual pulses. Each pulse is used for an incremental heating and/or for the measurement of opto-acoustic transients. The measured signal may be averaged over 5 to 25 or 50 measurements. The duration of the pulses may be controlled/adapted for a control of the heating or the tissue temperature. Hence, the laser pulses are controlled in the form of a pulse width modulation/ frequency modulation.

A shorter duration of the laser pulses in relation to the pulse pauses slows down the heating. This might be useful for heating the tissue close to treatment limit temperatures or for cooling the tissue slowly. Larger pulse durations may be used to heat up the tissue quickly. For a fast cooling, the pulse sequence may be turned off for a certain time.

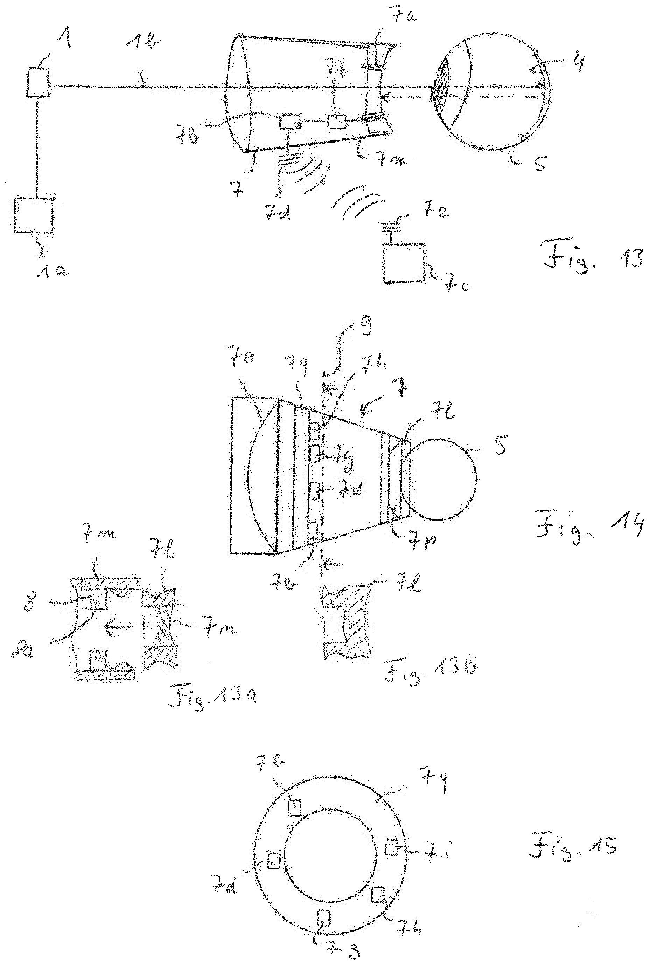

Figure 13 shows a setup of a laser treatment device with a measuring device for a control value, in particular a temperature measuring device, wherein the therapy laser 1 is emitting the laser beam 1b to the fundus 4 of the patient's eye and wherein the therapy laser is controlled by the control unit 1a in a way as to emit not only laser signals for the medical treatment and exclusively for purposes of heating the tissue at the fundus of the eye, but also dedicated laser signals which are used to detect pressure waves/transients which are generated by the dedicated laser signals. The detection of the pressure waves/transients happens in the same way as described with reference to figure 2 and using the same reference numbers for the same elements.

The control unit can be specially arranged to distinguish between laser signals which are intended just for heating and medical treatment of the tissue and dedicated laser signals that are intended to be used for determining a control value or specifically for temperature measurement. In the case that the control unit 1a provides a dedicated laser signal, it may send a signal to the temperature measuring device and align with this device, so that the (temperature) measuring device can use the dedicated signal and prepare for the measurement of the respective pressure wave/transient. The control unit may as well communicate with the measuring device/temperature measuring device about a time schedule of one, one to ten or one to fifty following dedicated laser signals. A data processing unit of the temperature measuring device may be combined with or integrated in the receiving unit 7c, so that the temperature or a control value may be finally calculated in this unit. A feedback loop 6 as shown in figure 1 between the receiving unit 7c and the control unit 1a may be provided to allow the control unit to control the therapy laser appropriately to reach and keep a target tissue temperature.

Figure 14 shows a cross section of a measuring unit for measuring pressure waves, which preferably may be implemented as a mobile unit 7. In the figure, the measuring unit is shown in mechanical contact with an eye 5. The mobile measuring unit 7 is pressed onto the eye with a certain pressure force for establishing good conditions to ensure that pressure waves are efficiently transported or transferred from the fundus through the eye body to the measuring unit and in the measuring unit to a pressure wave sensor/pressure converter.

One detail of the measuring unit 7 and its quality to ensure a good transmission or transfer of the pressure waves to the pressure wave sensor may be explained with reference to the figures 13a and 13b. Figure 13a shows an end part of the housing wall 7m of the measuring unit 7, wherein a pressure wave transfer body 8 is provided and fixed to the housing wall 7m.

The pressure wave transfer body 8 may also be formed by the housing wall 7 or be combined with it. The pressure wave transfer body 8 may contain or hold a pressure wave sensor/pressure converter in the form of any sensor which in this text is described as a potential implementation of such sensor. The Sensor may particularly be implemented as a piezo sensor 8a in the form of a ring of a piezo sensitive material which is fixedly mechanically connected to the pressure wave transfer body 8. The ring may as well only partially consist of a piezo sensitive material.

In figures 13a and 13b, a contact element 7l is shown which is provided for making direct contact to the patient's eye and transferring the pressure waves from the eye to the pressure wave transfer body 8. The measurement unit, which is used for different patients, either has to be cleaned and disinfected before use or the contact element can be implemented as a disposable contact element 7l or at least as an element which is separable from the housing of the measuring unit.

Figures 13a and 13b show a disposable contact element 7l which can be snapped in on the housing of the measuring unit 7 and has therefore a certain elasticity. The contact element 7l may be combined with a transparent cover 7n which covers the opening of the ring-shaped contact element, so that laser beams may pass through. In another implementation, the cover 7n and the contact element may be combined and made of the same transparent material as shown in figure 13b.

Figure 14 shows that the measuring unit 7 has a conical, tubular housing which at both ends is covered by a lens 7o, 7p. Instead of each of the lenses, on each side another optical element, like, e.g. flat glass or plastic, may be provided which is transparent at least for the laser beams used by the device. In one potential implementation, only one or no side of the tubular housing is covered by a transparent cover element.

In the tubular housing, at least one or two rings 7q may be provided and fixed to the inner side of the housing wall 7m. The ring 7q has an opening to let the laser beam pass through. On one or both front sides of the ring 7q, elements 7b, 7d, 7g,7g, 7h, 7i, 7j, 7k, of the preprocessing unit may be installed and the ring 7q itself may be implemented as a printed circuit board with printed or etched conductors.

Figure 15 is showing a front side view of the ring 7q as indicated by the dotted line 9 in figure 14.

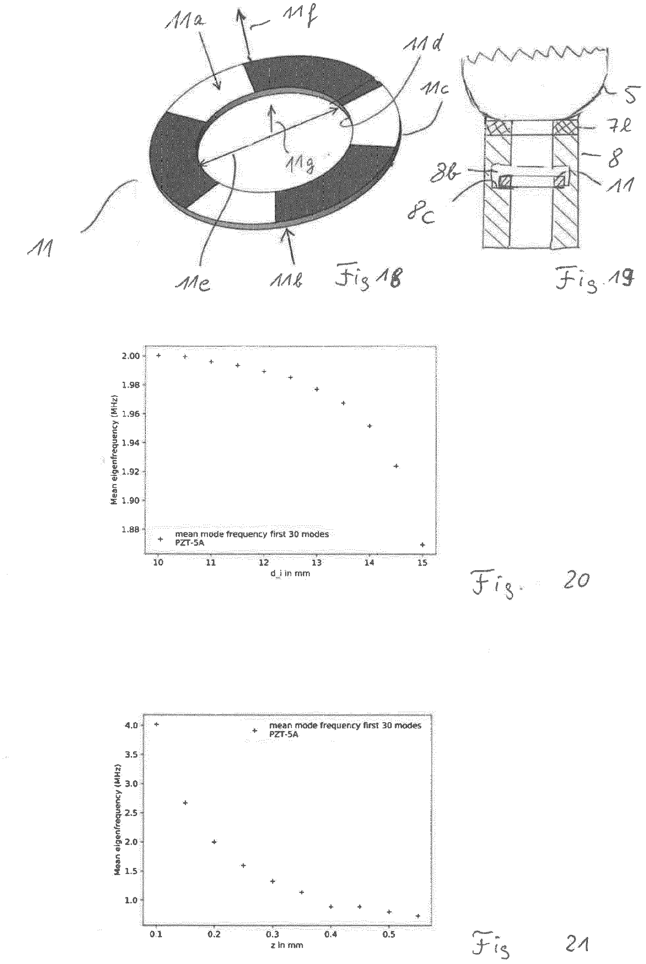

Further information about the pressure converter itself will be given with reference to figures 18ff below.

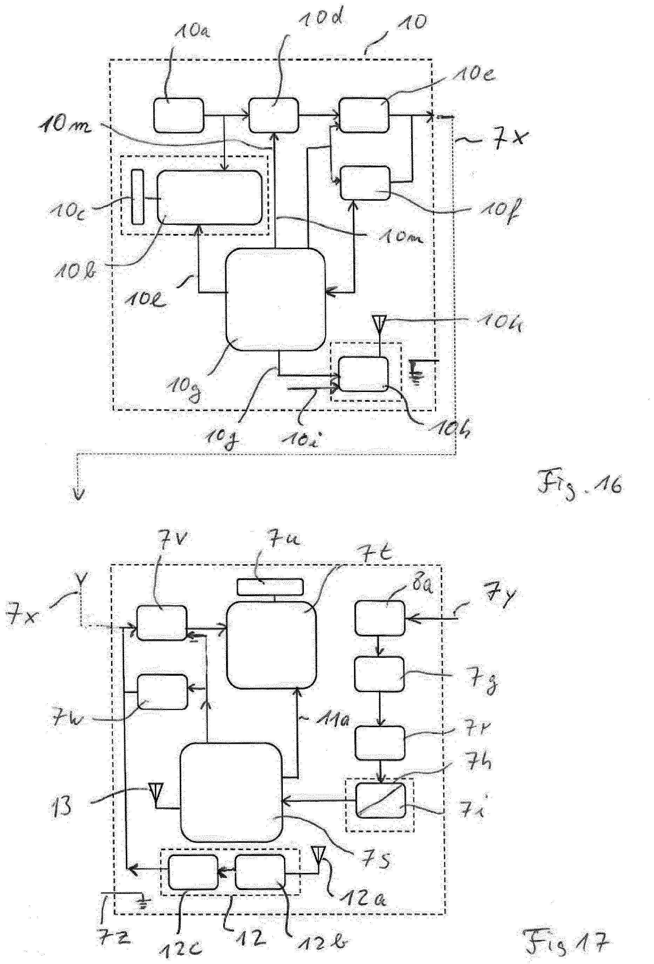

Figure 16 shows a detailed view of a charging station 10 for a mobile measuring unit of a temperature measurement device. In the figure, both possibilities are represented, a conducting contact between the charging station and the mobile measuring unit as well as a charging through a wireless interface like inductive charging which is indicated by an antenna 10k in figure 16. In reality, it is possible to realize in a charging station both or only one of the possibilities.

The charging station 10 comprises a USB connection 10b for power supply. From the USB connection, electric power is provided to a backup battery charger 10b which is connected to a backup battery 10c and charges the backup battery for emergency cases and the case of failure of the external power supply.

The USB connection is also connected to a voltage converter 10d which controls the voltage level of the voltage which is supplied to the mobile measuring unit. The voltage converter 10d is controlled by a pulse width modulation 10m which is provided by the microcontroller 10g. The microcontroller 10g also controls the backup battery charger 10b by a signal connection 10 l which makes use of a standardized communication protocol.

The microcontroller 10g also controls the two load switches 10e and 10f which both can establish or block the connection 7x to the mobile measuring unit 7, wherein load switch 10e controls the connection depending on the charging status while switch 10f has the function to protect the mobile measuring units in case of a short circuit or other kinds of failure.

The power line 7x establishes the electrically conductive power supply connection to the mobile measuring unit 7 in the case of charging. This hardware connection can for example be realized by metallic pressure contacts which may be spring loaded.

Figure 16 also shows an inductive field generator 10h which is controlled through a connection 10j by the microcontroller 10g, and which is connected to a power supply 10i. An antenna 10k is provided which can be realized by an inductive loop and which can couple with a respective loop in the mobile measuring unit in case of charging.

Figure 17 is showing a schematic view of a mobile measuring unit 7. The power supply of the unit 7 is provided by a rechargeable battery 7u. The battery is electrically connected to all elements of the unit 7 that need electric power supply, even if in the figure, the power supply connections are not shown for the sake of transparency of the drawing. The battery 7u is charged by a battery charger 7t which has the capacity to balance to a certain extent variable input voltages. The battery charger 7t is controlled by the microcontroller 7s through a signal line 11a which makes use of a standard protocol for the transfer of information.

The power supply of the battery charger 7t in case of charging can be provided through the power line 7x and alternatively through the inductive charging unit 12.

The power line 7x is connected to two load switches 7v, 7w of the mobile measuring unit, which can block or establish the electrical power supply connection between the power line 7x and the battery charger 7t.

The load switch 7v has the function to control the power supply connection from the functional perspective dependent on the charging status of the battery while load switch 7w can block the connection for protection reasons in case of failure.

In case the inductive charging unit 12 is used, the inductive loop 12a, which is symbolized as an antenna, is coupled with a respective loop 10k on the side of the charging station 10. A transformer 12b provides an ac power which is converted to a direct current and stabilized in the dc converter 12c. The dc converter 12 c is connected to the power line 7x and can supply power to the unit 7 in the same way as the hardware connection.

The mobile measuring unit on the measuring side comprises a pressure converter 8a, which can be a piezo converter, an interferometric converter or a pressure converter of any other kind mentioned in this text. The pressure converter 8a receives pressure waves through a pressure wave transfer element which might be a pressure wave transfer body 8 as shown in 13a or in figures 18ff. The transfer of the pressure waves in figure 17 is symbolized by the reference number 7y.

The pressure converter is converting transient pressure wave signals into electrical signals. These signals are supplied to a filter 7g which can be a high pass filter, a low pass filter or a band pass filter. The filtered signal is then supplied to an amplifier 7r which may e.g., have an amplification factor of 40-60dB.

The amplified signals are supplied to a signal integrator 7h and further to a signal averaging unit 7i, where the average over several signals can be calculated. The calculated values can then be sent to the microcontroller 7s and further sent via a wireless connection using the antenna 13, to a stationary receiving unit which can display a tissue temperature and send temperature values or control values to the controller of a therapy laser.

Between the microcontroller 7s and the analog switch 7w a connection line is established which is arranged to transmit signals according to a communication protocol as for example UART TX allowing for a comfortable, potentially bidirectional exchange of information.

As described above, in many cases for the detection and measuring of pressure waves, a ring-shaped piezo sensor is used. Such a ring is completely or at least partially made of a material with piezo effect, i.e., it develops an electric voltage signal as soon as a pressure on the material is applied or changed.

One advantage of piezo pressure converters is that they react very fast on pressure changes and hence can convert pressure changes of very high frequency.

One example of a ring-shaped piezo pressure converter or pressure transducer is shown in figure 18 in a perspective view. The ring 11 of figure 18 has a circular shape and a rectangular cross section. It has a first front side 11a which is also called the top side and which in use faces the eye of a patient, a second front side 11b which is also called the bottom side, an inner circumferential side (inner rim) 11d and an outer circumferential side (outer rim) 11c. The inner diameter of the ring is referenced with the reference sign 11e. The arrow 11f points into the direction of the eye of the patient, when the pressure converter is in use and fixed with its bottom side 11b to a pressure transfer body.

The thickness of the ring 11 is measured in its axial direction which is represented by the arrow 11g.

In figure 19, the ring 11 of figure 18 is shown in connection with a hollow cylindrical pressure wave transfer body 8. The eye of the patient has the reference sign 5. The pressure wave transfer body has a ring shaped opening or groove 8b with a rectangular cross section running around its inner circumference. The ring 11 is positioned in the opening 8b and fixedly connected to the bottom 8c of the opening 8b which is farther away from the eye. The ring can, e.g., be fixed with its second front side 11b (the bottom side) to the material of the pressure wave transfer body by an adhesive. The other three sides of the ring are not connected to the pressure wave transfer body. The outer circumferential side 11c as well as the first front side 11a of the ring have a distance to the material of the body 8. This distance can be left empty or filled by a decoupling, soft substance like a foam.

A pressure wave which is generated by an opto-acoustic pulse at the fundus of the eye 5 will travel through the eye and through the disposable contact element 7l and into the pressure wave transfer body 8. The pressure wave transfer body transfers the pressure waves to the bottom side 11b of the pressure converter 11 and the pressure converter vibrates and converts the vibration into electric signals.

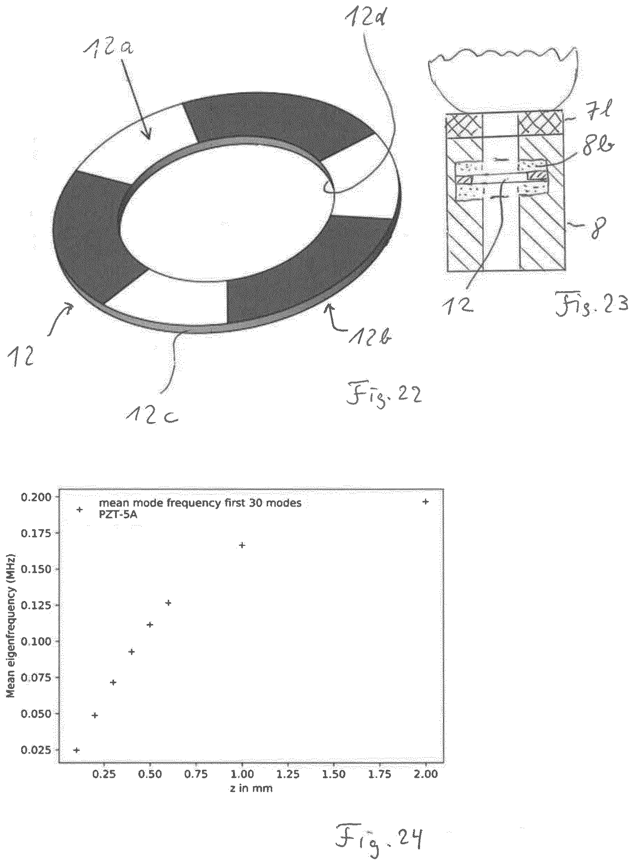

Figure 20 shows in a diagram the relation between the mean eigenfrequency of the ring 11 and the inner diameter of the ring which is shown in figure 18 for the case that its bottom side 11b is connected to the pressure wave transfer body 8 according to fig.19. As also for the diagrams of figures 21 and 24, the mean eigenfrequency has been calculated as the mean mode frequency of the first 30 vibrational modes of the ring 11.

It can be seen that the relation between the mean eigenfrequency and the inner diameter of the ring is clear, monotone and there is not a big change of the eigenfrequency if the inner diameter of the ring is changed between 10 and 15 mm. It is evident how the eigenfrequency of the ring can be changed by varying the inner diameter of the ring as long as the ring is fixed to the pressure wave transfer body 8 with the one of its front sides which during use is facing away from the patient's eye.

Figure 21 shows the relation between the mean eigenfrequency of the ring and the thickness of the ring as shown in figure 18 measured in its axial direction in case its bottom side of the ring facing away from the patient's eye is connected to a pressure wave transfer body as shown in fig.19.

It can be seen from figure 21 that the relation between the mean eigenfrequency of the ring 11 and the thickness of the ring is also transparent and clear. The mean eigenfrequency of the ring can be changed in a relatively wide range by varying the thickness of the ring between 0,1 mm and 0,5 mm. Would the ring be connected to the pressure wave transfer body in another way, e.g. by a fixed mechanical connection of more than one of its sides, then the relations as shown in figs.20 and 21 would be more complex, maybe not monotone, difficult to predict and an alignment or relative tuning of laser characteristics and the geometry and size of the pressure converter ring to one another would become way more difficult.

Figure 22 shows a perspective view of a pressure converter in the form of a piezo ring 12, which shall be at its outer circumferential side 12c fixedly connected by an adhesive to a pressure wave transfer body 8. The piezo ring 12 further has a first front side 12a, a second front side 12b and an inner circumferential side 12d.

Figure 23 shows that the pressure wave transfer body 8 has, as already shown in figure 19, a ring-shaped opening or groove 8b with a rectangular cross section which is running in azimuthal direction around its inner circumference.

The outer rim or outer circumferential side 12b of the ring 12 is either clamped into the groove 8b or connected to the bottom of the groove 8b by an adhesive. The two font sides 12a and 12b of the ring 12 as well as the inner circumferential side 12d are not touching the pressure wave transfer body 8. On the sides 12a, 12b, a distance is provided between the material of the ring and the pressure wave transfer body 8. The distance can be left open or be filled with a decoupling material like a foam or gel. This material can also be chosen to form a damping layer for the ring which can be used to fine-tune the mean eigenfrequency.

In figure 23, the snap-in connection between the pressure wave transfer body 8 and the disposable contact element 7l is not shown but the connection my have a form analog to the connection shown in figure 13a or it can work with any other form of snap-in connection.

Figure 24 shows the relation between the mean eigenfrequency of the ring 12 and the thickness of the ring as shown in figure 18 in its axial direction in case the rim/outer circumference side of the ring 12 is connected to a pressure wave transfer body 8 as is shown in figure 23. It can be seen that there is a clear and monotone relation between the thickness z of the ring, measured in its axial direction, and the mean eigenfrequency. The variation of the thickness of the ring can be used in a predictable, transparent way to tune the mean eigenfrequency of the ring to characteristics of an excitation laser which is delivering the pulses for the temperature measurement or the measurement or determination of a control value.

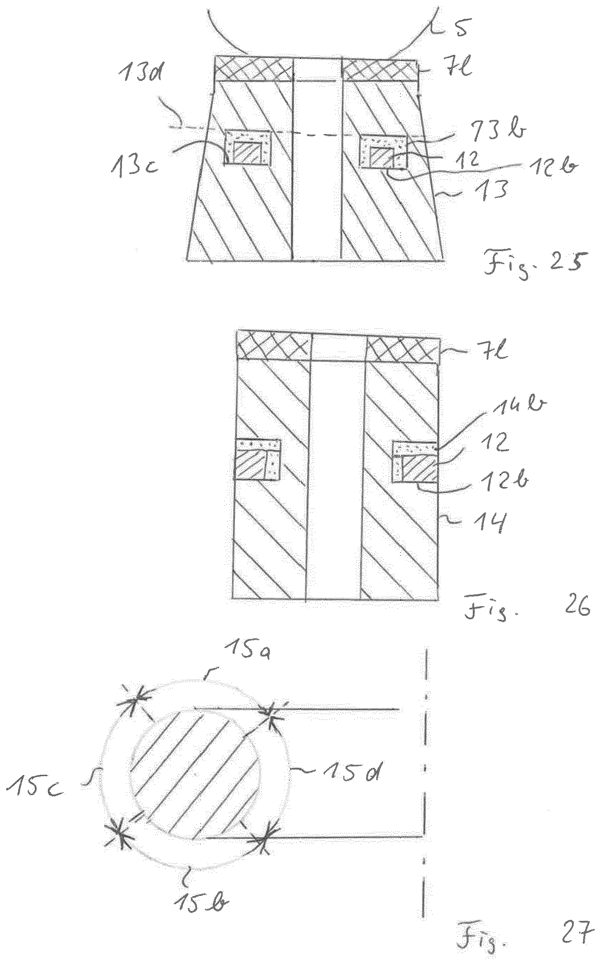

Figure 25 shows a cross sectional view of a pressure wave transfer body 13 of conical outer shape with a central cylindrical bore. A ring-shaped hollow space 13b of rectangular cross section is provided inside the pressure wave transfer body and in the hollow space, a pressure converter ring is positioned. The bottom side (second front side) 12b of the pressure converter is fixedly connected to the bottom 13 c of the hollow space, i.e., with the pressure wave transfer body. Each of the other sides of the pressure converter 12 is facing the material of the pressure wave transfer body with a distance which can be filled by a foam. The foam can protect the pressure converter from undesired influences. The pressure wave transfer body may be separable along the dotted line 13d and may consist of two parts which can be taken apart for introducing the pressure converter 12.

At the front end of the pressure wave transfer body, a disposable contact element 7l is provided which makes contact to the eye 5 of a patient.

Figure 26 shows a cross sectional view of a hollow cylindrical pressure wave transfer body 14 with an opening or groove 14b running in azimuthal direction around its outer circumferential side. The groove has a rectangular cross section and a pressure converter ring 12 is positioned in the groove. A bottom side (second front side) 12b of the pressure converter is fixedly connected to the pressure wave transfer body 14. The inner rim/inner circumferential side 12d of the pressure converter as well as its first front side have a distance to the material of the pressure wave transfer body. The distance may be filled with a foam in order to protect the pressure converter from environmental influences.

The pressure converter 12 has electrodes for deducting the voltage signal which is generated by the pressure changes. The electrodes are not shown in the figures and the setup is well known.

Figure 27 shows a cross sectional view of a pressure converter in the form of a toroid 15, i.e., a ring with a circular cross section. In this case of a ring of a not rectangular but circular cross section, the first front side 15a and the second front side 15bc, the inner circumferential side 15d and the outer circumferential side 15c of the ring 15 may be defined each as a surface section of 20-30% or an angular section of about 90 degrees of the circumference of the ring in the cross-sectional view as shown in the figure 27.


**Claims**

1. 1. Measuring device for measuring a control value, in particular the tissue temperature in the eye (5) of a patient with an excitation source (1, 2) for pulse-like excitation of the tissue by means of a beam falling through the eye lens, in particular a laser beam (1b, 2b) or laser signal, with a mobile measuring unit (7) for placing on the patient's eye for the detection and/or measurement of opto-acoustic pulses (16a, 16b) or pressure waves and with a receiving unit (7c) for receiving data from the mobile measuring unit, wherein the mobile measuring unit comprises a pressure converter (7a, 8a, 11, 12) for measuring transient pressure signals, a signal pre-processing unit (7f) and a data transmitting unit (7b) for wireless data transmission and wherein the receiving unit (7c) is arranged for the wireless reception of data.

2. Treatment device for the treatment of biological tissue, in particular tissue in the eye of a patient, by radiation, with a radiation source, in particular a therapy laser (1), for targeted heating of the tissue with the aim of generating a tissue reaction, and with a measuring device, in particular a temperature measuring device, according to claim 1.

3. Treatment device according to claim 2, **characterized in that** the excitation source (1, 2) is identical to the radiation source, in particular a therapy laser (1).

4. Mobile measuring unit for a measuring device according to claim 1 for placing on the patient's eye (5), which comprises a pressure converter (7a, 8a, 11, 12) for detecting transient pressure wave signals and a signal preprocessing unit (7f), a data transmitting unit (7b) for wireless data transmission and an antenna (7d, 7e, 7k).

5. Mobile measuring unit according to claim 4, wherein the signal preprocessing unit (7f) comprises at least one, two, three, four, five, six or seven of the following elements: an amplitude modulator or a frequency modulator for modulating the measured signal on a radio wave, a signal amplifier (7r), a band pass filter, a high pass filter, a low pass filter (7g), a unit (7j) for digitizing data or signals, a unit (7h) for integrating over signals, a unit (7i) for averaging over signals or data.

6. Mobile measuring unit according to claim 4 or 5, **characterized by** rechargeable energy storage means (7u).

7. Mobile measuring unit according to claim 4, 5 or 6, **characterized by** a device (7s,7v, 7w) for overvoltage protection and/or for short-circuit protection and/or for protection against incorrect polarity.

8. Mobile measuring unit according to claim 4, 5, 6 or 7, **characterized by** a disposable contact element (7l, 7n) for establishing the physical contact to the patient's eye (5), which is or can be fixed on a front side of the tubular housing (7m) or of a pressure wave transfer body (8) by a snap-in mechanism in such a way that it is in steady contact with a carrier of the pressure converter, in particular a pressure wave transfer body.

9. Mobile measuring unit according to one of claims 4 to 8, **characterized in that** one, two, three or more than three of the following elements are placed inside a tubular housing of the mobile measuring unit: a signal preprocessing unit (7f), a data transmitting unit (7b), a device for overvoltage protection, a device for short-circuit protection, a device for protection against incorrect polarity (7s), a rechargeable energy storage means (7u).

10. Mobile measuring unit according to one of claims 4 to 9 with a tubular housing (7m), **characterized in that** an antenna (7d, 7k) for data transmission is provided inside the tubular housing or on the outside of the tubular housing of the mobile measuring unit or in an opening of the tubular housing or that an antenna is formed by an opening of the tubular housing, wherein the antenna is connected to the data transmitting unit (7b).

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 13a

Fig. 13b

Fig. 14

Fig. 15

Fig. 16

Fig 17

Fig 18

Fig. 19

Fig. 20

Fig 21

Fig. 22

Fig. 23

Fig. 24

13d

5

7l

73b

13c

12    12b

13

Fig. 25

7l

14b

12

12b

14

Fig. 26

15a

15c

15d

15b

Fig. 27

Europäisches
Patentamt

European
Patent Office

Office européen
des brevets

# EUROPEAN SEARCH REPORT

Application Number

EP 22 21 3252

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/245769 A1 (ZHANG HAO F [US] ET AL) 30 September 2010 (2010-09-30) | 1 | INV.<br>A61F9/008<br>A61B5/00 |
| Y | * figure 1 *<br>* paragraphs [0023], [0025], [0074] * | 2,3 | |
| Y | EP 1 279 385 A1 (MED LASERZENTRUM LUEBECK GMBH [DE]) 29 January 2003 (2003-01-29)<br>* paragraphs [0001], [0020], [0023], [0026], [0029] * | 2 | |
| Y | DE 10 2021 201080 B3 (MEDIZINISCHES LASERZENTRUM LUEBECK GMBH [DE]) 7 April 2022 (2022-04-07)<br>* paragraph [0015] * | 3 | |
| X | US 8 939 906 B2 (UNIV NAT CHIAO TUNG [TW]) 27 January 2015 (2015-01-27) | 4,5 | |
| Y | * column 1, line 56 – column 2, line 8 *<br>* figure 1 * | 7,8 | |
| X | US 2009/076367 A1 (SIT ARTHUR J [US] ET AL) 19 March 2009 (2009-03-19)<br>* paragraphs [0039], [0059], [0060], [0064], [0065] *<br>* figure 12 *<br>* claim 24 * | 4,6,9,10 | **TECHNICAL FIELDS SEARCHED (IPC)**<br><br>A61F<br>A61B |
| Y | US 2005/231686 A1 (RATHJEN CHRISTIAN [DE]) 20 October 2005 (2005-10-20)<br>* paragraphs [0013], [0051] *<br>* figure 5b * | 8 | |
| Y | CN 109 452 929 A (SHENZHEN HUAZHIKANG ELECTRONICS CO LTD) 12 March 2019 (2019-03-12)<br>* paragraphs [0005] – [0008] * | 7 | |

The present search report has been drawn up for all claims

| Place of search | Date of completion of the search | Examiner |
|---|---|---|
| Munich | 26 July 2023 | Jansen, Birte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

**EP 22 21 3252**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

The Search Division considers that the present European patent application does not comply with the requirements of unity of invention and relates to several inventions or groups of inventions, namely:

```
1. claims: 1-3

        Device including a therapeutic laser.
                        ---


2. claims: 1, 4-10

        Device including an antenna.
                        ---
```

## ANNEX TO THE EUROPEAN SEARCH REPORT
## ON EUROPEAN PATENT APPLICATION NO.

EP 22 21 3252

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2023

| Patent document cited in search report | | Publication date | Patent family member(s) | | Publication date |
|---|---|---|---|---|---|
| US 2010245769 | A1 | 30-09-2010 | EP 2408376 | A1 | 25-01-2012 |
| | | | US 2010245766 | A1 | 30-09-2010 |
| | | | US 2010245769 | A1 | 30-09-2010 |
| | | | US 2010245770 | A1 | 30-09-2010 |
| | | | US 2010249562 | A1 | 30-09-2010 |
| | | | WO 2010107930 | A1 | 23-09-2010 |
| | | | WO 2010107933 | A1 | 23-09-2010 |
| EP 1279385 | A1 | 29-01-2003 | DE 10135944 | A1 | 20-02-2003 |
| | | | EP 1279385 | A1 | 29-01-2003 |
| | | | US 2003032949 | A1 | 13-02-2003 |
| DE 102021201080 | B3 | 07-04-2022 | DE 102021201080 | B3 | 07-04-2022 |
| | | | WO 2022167562 | A1 | 11-08-2022 |
| US 8939906 | B2 | 27-01-2015 | NONE | | |
| US 2009076367 | A1 | 19-03-2009 | US 2009076367 | A1 | 19-03-2009 |
| | | | WO 2007136993 | A1 | 29-11-2007 |
| US 2005231686 | A1 | 20-10-2005 | AT 261261 | T | 15-03-2004 |
| | | | AU 2003240367 | A1 | 19-01-2004 |
| | | | EP 1374758 | A1 | 02-01-2004 |
| | | | US 2005231686 | A1 | 20-10-2005 |
| | | | WO 2004002299 | A1 | 08-01-2004 |
| CN 109452929 | A | 12-03-2019 | NONE | | |

EPO FORM P0459

For more details about this annex : see Official Journal of the European Patent Office, No. 12/82

**EP 4 385 470 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10135944 C2
- US 20030032949 A1